# EUROPEAN PATENT APPLICATION

(11) **EP 3 812 396 A1**
(43) Date of publication of application: **28.04.2021**
(21) Application number: 19204386.7
(22) Date of filing: 21.10.2019
(51) Int. Cl.: C07K 14/575

(54) **METHODS FOR TREATING AND/OR PREVENTING HYPERSOMNIAS**

(71) Applicant: Université de Lausanne, 1011 Lausanne (CH)
(72) Inventor: TAFTI, Mehdi, 1224 Chêne-Bougeries (CH); SEIFINEJAD, Ali, 3007 Bern (CH)
(74) Representative: KATZAROV S.A.

(57) **Abstract**

Compounds, compositions, and methods for treating and/or preventing a neurological disease, a neuropsychiatric disease, a neurodegenerative disease, preferably narcolepsy and associated diseases or disorders.

SEQUENCE LISTING

<110>  Université de Lausanne

<120>  Methods for treating and/or preventing hypersomnias

<130>  52091/EP

<160>  17

<170>  PatentIn version 3.5

<210>  1
<211>  608
<212>  DNA
<213>  Homo sapiens

<400>  1
   agctaggggg agggggcagc taaggagcct ttccatgaag gaagaaggtc ctggagcctg      60
   acagtcccca ggagcagcga caagaagcag gggagggaga ggactgctgc tggctgctcc     120
   accccccaca cacataatgt ggggtctcgc gtctgcctct ctcccgcccc taattagcag     180
   ctgcctccct ccatattgtc ccaggccagc gcctcttttg tgctcccaga ttcctgggtg     240
   caaggtggcc tcattagtgc ccggagaccg ccccatctcc agggagcaga tagacagaca     300
   agggggtgat caggggcaca gtgatccaac cctggcctct gaacgccgca gcggccattc     360
   cttgggccca gcctggagac ggcccccctg cagcaggcta atcttagact tgcctttgtc     420
   tggcctgggt gtggacgcaa gtgcctgtca attccccgcc acctcagagc actataaacc     480
   ccagacccct gggagtgggt cacaattgac agcctcaagg ttcctggctt tttgaaccac     540
   cacagacatc tcctttcccg gctaccccac cctgagcgcc agacaccatg aaccttcctt     600
   ccacaaag                                                              608


<210>  2
<211>  26
<212>  DNA
<213>  Unknown

<220>
<223>  synthetic sequence

<400>  2
gaagaaggtt ttggagtttg atagtt                                           26


<210>  3
<211>  24
<212>  DNA
<213>  Unknown

<220>
<223>  synthetic sequence

<400>  3
   ctatacccct aatcaccccc ttat                                              24

<210>  4
<211>  25
<212>  DNA
<213>  Unknown

<220>
<223>  synthetic sequence

<400>  4
ttgggtgtaa ggtggtttta ttagt                                             25


<210>  5
<211>  25
<212>  DNA
<213>  Unknown

<220>
<223>  synthetic sequence

<400>  5
ttataaccca ctcccaaaaa tctaa                                             25


<210>  6
<211>  25
<212>  DNA
<213>  Unknown

<220>
<223>  synthetic sequence

<400>  6
ggattgttgt tggttgtttt atttt                                             25


<210>  7
<211>  24
<212>  DNA
<213>  Unknown

<220>
<223>  synthetic sequence

<400>  7
   ctatacccct aatcaccccc ttat                                             24


<210>  8
<211>  24
<212>  DNA
<213>  Unknown

<220>
<223>  synthetic sequence

<400>  8
ataagggggt gattaggggt atag                                              24


<210>  9

<211>  25
<212>  DNA
<213>  Unknown

<220>
<223>  synthetic sequence

<400>  9
ttataaccca ctcccaaaaa tctaa                                              25


<210>  10
<211>  25
<212>  DNA
<213>  Unknown

<220>
<223>  synthetic sequence

<400>  10
gttgtgtttt tgttagggtt agtgt                                              25


<210>  11
<211>  27
<212>  DNA
<213>  Unknown

<220>
<223>  synthetic sequence

<400>  11
caaaaactaa aacaatcttc taatcac                                            27


<210>  12
<211>  30
<212>  DNA
<213>  Unknown

<220>
<223>  synthetic sequence

<400>  12
gtttggtatt agtttaggta tgtattagag                                         30


<210>  13
<211>  27
<212>  DNA
<213>  Unknown

<220>
<223>  synthetic sequence

<400>  13
caaaaactaa aacaatcttc taatcac                                            27


<210>  14
<211>  26
<212>  DNA
<213>  Unknown

<220>
<223>  synthetic sequence

<400>  14
ggtgttattg ttgtagtttt tagtgt                                            26


<210>  15
<211>  24
<212>  DNA
<213>  Unknown

<220>
<223>  synthetic sequence

<400>  15
aactcttaaa tttcattcaa ctcc                                              24


<210>  16
<211>  25
<212>  DNA
<213>  Unknown

<220>
<223>  synthetic sequence

<400>  16
gaggtattgg tttagtaatt tttta                                             25


<210>  17
<211>  24
<212>  DNA
<213>  Unknown

<220>
<223>  synthetic sequence

<400>  17
aactcttaaa tttcattcaa ctcc                                              24

## Description

### FIELD OF THE INVENTION

Compounds, compositions, and methods for treating and/or preventing a neurological disease, a neuropsychiatric disease, a neurodegenerative disease, preferably narcolepsy and associated diseases or disorders.

### BACKGROUND OF THE INVENTION

Narcolepsy with cataplexy (also called narcolepsy type 1 or NT1) is a sleep disorder characterized by excessive daytime sleepiness and cataplexy (loss of skeletal muscle tone triggered by strong emotions). The best biological marker of the disease is hypocretin deficiency (CSF HCRT-1 levels < 40pg/ml) (Nishino et al., 2000). Post-mortem examination of the hypothalamic region of narcolepsy patients indicated a near absence of hypocretin neurons (Peyron et al., 2000). Together with strong associations between narcolepsy and HLA-DQB1*06:02 allele (Tafti et al., 2014) and a T cell receptor alpha (Hallmayer et al., 2009) and the acute increase in childhood narcolepsy following the 2019-2010 H1N1 vaccination (Dauvilliers et al., 2010), it is widely accepted that an immune or autoimmune process leads to hypocretin neuronal loss. Also recently, autoreactive T cells were reported against various antigens (Latorre et al., 2018). Nevertheless, a direct evidence of hypocretin neuronal damage (dying cells or local lateral hypothalamic inflammation) is still lacking.

Hypocretin deficiency can be caused by either neuronal death or absence of hypocretin production. Evidence in patients with secondary narcolepsy (associated with Wernicke's encephalopathy (Kashiwagi M, 2004), multiple sclerosis (Oka et al., 2004), anti-aquaporin 4 antibody (Nozaki et al., 2009), or tumors (Dauvilliers et al., 2007)) indicate that the CSF HCRT-1 can normalize following successful treatment, strongly suggesting that hypothalamic lesions or inflammation may lead to reversible HCRT deficiency. More interestingly, the Inventors have reported on a 28 year old female patient diagnosed with narcolepsy with cataplexy and hypocretin deficiency 15 days after the first cataplexy attack, that has been treated with IVIg and her CSF HCRT-1 level normalized together with her symptoms (Dauvilliers et al., 2009).

Despite the advances that have been made in this field, a need remains in the art for improved compounds, compositions, and methods for treating and/or preventing a neurological disease, a neuropsychiatric disease, a neurodegenerative disease, preferably narcolepsy and associated diseases or disorders. The present disclosure fulfills these and other needs, as evident in reference to the following disclosure.

### SUMMARY OF THE INVENTION

The present invention provides an agent for use in the treatment and/or prevention of narcolepsy, wherein the agent modulates the level of methylation of at least one methylation site within the 5 ' region upstream from the transcription start site of the human hypocretin neuropeptide precursor (HCRT) gene.

Also provided is a plasmid or a vector comprising one or more nucleic acid(s) encoding the miRNA, siRNA, piRNA, hnRNA, snRNA, sgRNA, esiRNA, shRNA, and/or antisense oligonucleotide of claim 10.

Further provided is a host cell comprising a plasmid or vector of claim 11 or one or more nucleic acid(s) encoding the miRNA, siRNA, piRNA, hnRNA, snRNA, sgRNA, esiRNA, shRNA, and/or antisense oligonucleotide of the invention.

Further provided is a pharmaceutical composition comprising a therapeutically effective amount of i) an agent modulating the level of methylation of at least one methylation site within the 5 ' region upstream from the transcription start site of the human hypocretin neuropeptide precursor (HCRT) gene, or ii) a plasmid or a vector of the invention, or iii) a host cell of the invention, and a pharmaceutically acceptable carrier or diluent.

### BRIEF DESCRIPTION OF THE FIGURES

**Fig. 1****.** The first 500bp sequence of the proximal hHCRT promoter upstream of the first exon (transcription start site, in capital italic) and the cDNA (in capital bold), SEQ ID NO. 1. CpGs are highlighted in bold. A critical Pax5-Ets1 binding site (Pax5-EBS) is boxed and the TATA box is in italic and underlined.
**Fig. 2****.** Methylation of the 12 CpGs of the *hHcrt* promoter. Data are from single molecule deep sequencing of 2 overlapping PCR fragments (percentage + sem, n=7 in each group).
**Fig. 3****.** Methylation of 10 CpGs of the *hPDYN* proximal promoter. Data are from single molecule deep sequencing of a single PCR fragment (percentage + sem, n=5 in narcoleptics and n=6 in controls).
**Fig. 4****.** Luciferase assay of the *hHcrt* promoter. Values are average of 3 wells measured in duplicate over 3 different transfections (n=9/vector) reported as a percent of the reference unmethylated vector. Error bars=1sd.

### DESCRIPTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

Reference throughout this specification to "one aspect", "an aspect", "another aspect", "a particular aspect", "combinations thereof" means that a particular feature, structure or characteristic described in connection with the invention aspect is included in at least one aspect of the present invention. Thus, the appearances of the foregoing phrases in various places throughout this specification are not necessarily all referring to the same aspect. Furthermore, the particular features, structures, or characteristics may be combined in any suitable manner in one or more aspects.

The term "comprise(s)", or "comprising" is generally used in the sense of include(s)/including, that is to say, permitting the presence of one or more features or components. The terms "comprise(s)" and "comprising" also encompass the more restricted ones "consist(s)" and "consisting", respectively.

As used in the specification and claims, the singular form "a", "an" and "the" include plural references unless the context clearly dictates otherwise.

As used herein, "one or more" agent(s)/compound(s) means "at least one" agent or compound, e.g. a combination of two, three, four, five, six, *etc...* agents/compounds.

The term "treatment and/or prevention" or "treating and/or preventing" means any administration of a composition, pharmaceutical composition, agent, compound, etc... of the disclosure to a subject for the purpose of: (i) preventing the disease, that is, causing the clinical symptoms of the disease not to develop; (ii) inhibiting the disease, that is, arresting the development of clinical symptoms; and/or (iii) relieving the disease, that is, causing the regression of clinical symptoms.

In the context of the present invention, the disease is selected from the group comprising, or consisting of, a neurological disease, a neuropsychiatric disease, a neurodegenerative disease, and associated diseases or disorders. Non-limiting examples of neurological diseases are usually selected from the group comprising, or consisting of, neuroinflammatory diseases (such as e.g. multiple sclerosis, traumatic brain injury, autoimmune neuroinflammatory of the CNS and spinal cord) and hypersomnias of central origin. Preferably, the neurological disease is hypersomnia, most preferably narcolepsy, more preferably narcolepsy with cataplexy also called narcolepsy type 1 or NT1.

Neurobehavioral disorders are selected from the group comprising, or consisting of, autism spectrum, fragile X syndrome, ADHD, traumatic brain injury, or a combination thereof.

Neurodegenerative diseases are selected from the group comprising, or consisting of, Alzheimer's disease, Parkinson's disease, Huntington's disease, Spinocerebellar ataxia and Amyotrophic Lateral Sclerosis or a combination thereof.

Neuropsychiatric diseases are selected from the group comprising, or consisting of, Schizophrenia, depression, bipolar disease, anxiety, or a combination thereof.

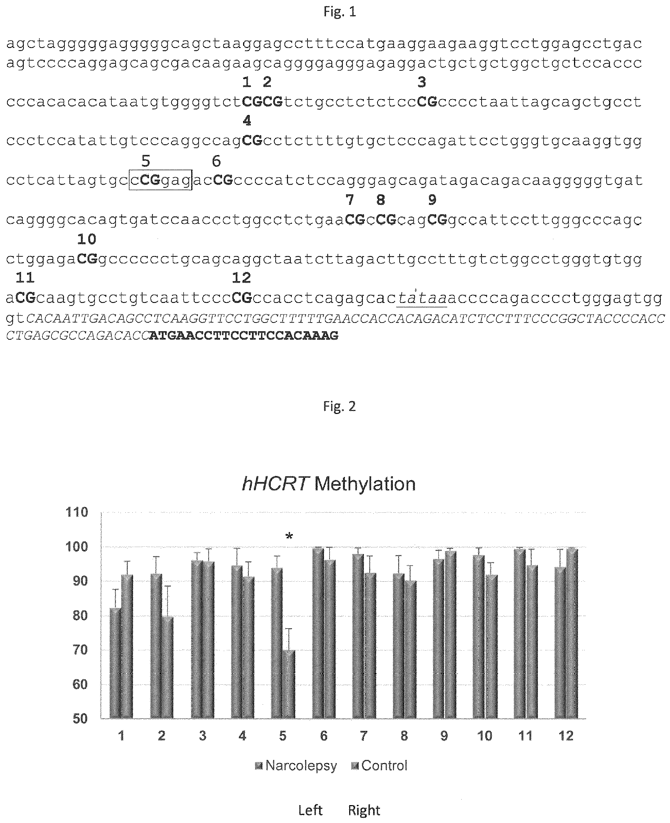

The present invention is based, in part, on the surprising results showing that an epigenetic silencing instead of HCRT neuronal loss causes narcolepsy. Epigenetic mechanisms include DNA methylation, histone modifications, chromatine structure and microRNAs. DNA methylation and histone modifications are the major mechanisms for gene silencing. The Inventors have evaluated the promoter methylation of HCRT in DNA extracts from lateral hypothalami of 7 narcolepsy with cataplexy and 7 control subjects. As shows in Fig. 1 and the Examples, the human HCRT promoter contains 13 CpGs. Twelve of the 13 CpG sites (all except the first CpG in Fig. 1) were tested. Eight of the 12 CpGs showed higher methylation in narcoleptics and one was significantly hypermethylated.

An aspect of the invention concerns an agent for use in the treatment and/or prevention of a neurological disease, a neuropsychiatric or neurobehavioral disease, a neurodegenerative disease, and associated diseases or disorders, preferably narcolepsy and hypersomnias, wherein the agent modulates the level of methylation of at least one methylation site within the 5 ' region upstream from the transcription start of the hypocretin neuropeptide precursor (HCRT) gene. Preferably, the agent decreases the level of methylation of said at least one methylation site within the 5 ' region upstream from the transcription start site of the human hypocretin neuropeptide precursor (HCRT) gene.

Usually, the 5 ' region upstream from the transcription start site of the human hypocretin neuropeptide precursor (HCRT) gene comprises one or more regulatory elements associated with said HCRT gene. Preferably, said at least one methylation site is in a CpG region located between base pairs -238 and -239 upstream of the transcription start site of the human hypocretin neuropeptide precursor (HCRT) gene. Most preferably, the CpG region is in a Pax5-Ets1 binding site (ccggag).

In one aspect, the agent of the invention is selected from the group comprising, or consisting of, a chemical compound, a peptide or analog or derivative thereof, an antibody or an antigen-binding fragment of said antibody and a nucleic acid, or a combination thereof.

As used herein, a "chemical compound" is a compound that produces change by virtue of its chemical composition and its effects on living tissues and organisms. The chemical compound may be a small molecule inhibitor (SMI) of DNA methylation or possesses DNA-demethylating properties and is preferably a non-peptidyl molecule.

Preferably, the non-peptidyl chemical compound possessing DNA methylation inhibition or DNA-demethylating properties is selected from the group comprising, or consisting of, nucleoside DNMT inhibitors (such as e.g. 5-azacytidine (Vidaza), 5-aza-2'-deoxycytidine (Decitabine or Dacogen), 5-fluoro-2'-deoxycytidine, and zebularine), non-nucleoside inhibitors of DNMT, Hydrazinophthalazines-derivates (hydralazine and dihydralazine), polyphenol (-)-epigallocatechin-3-gallate (EGCG), and procainamide, or a combination thereof as well as those described in Hauser et al., 2018. Most preferably, the small molecule inhibitor is a DNA methyltransferase inhibitor.

Methods for testing the DNA-demethylating properties of a compound (non-peptidyl chemical compound, peptide or nucleic acid) are well known in the art and include those described in de la Cruz-Hernandez et al., 2011 or Pérez-Cárdenas et al., 2018.

As used herein, the terms "peptide", "protein", "polypeptide", "polypeptidic" and "peptidic" are used interchangeably to designate a series of amino acid residues connected to the other by peptide bonds between the alpha-amino and carboxy groups of adjacent residues.

The term "derivative" of a peptide of the invention shall be taken to mean a peptide that is derived from an inhibitory peptide of the invention as described herein e.g., a fragment or processed form of the peptide, wherein the active portion of the base peptide is not modified e.g., a functional fragment.

The term "analog", as used herein, shall be taken to mean a peptide wherein the active portion is modified e.g., to comprise one or more naturally-occurring and/or non-naturally-occurring amino acids, provided that the peptide analog is, e.g., capable of preventing partially or completely the interaction between the at least one methylation site within the 5' region upstream from the transcription start of the hypocretin neuropeptide precursor (HCRT) gene and a DNA methyltransferase. For example, the term "analog" encompasses an inhibitory peptide comprising one or more conservative amino acid changes. In another example, an "analog" comprises one or more D-amino acids, a combination of D- and L-amino acids, and various unnatural amino acids (e.g., α-methyl amino acids, Cα-methyl amino acids, and Nα-methyl amino acids, etc) to convey special properties) known in the art. In this respect, such a retroinverso peptide analog may optionally include an additional component, such as, for example, a protein transduction domain, which may also be retroinverted.

Preferably, the peptide, analog or derivative thereof of the invention is able to modulate epigenetic mechanisms. The peptide, analog or derivative can directly interact with DNA in the at least one methylation site within the 5' region upstream from the transcription start of the hypocretin neuropeptide precursor (HCRT) gene, causing strand separation and initiation of gene transcription. These peptides, analog or derivative thereof are usually comprised of four amino acids and are called short peptides (see e.g. Janssens et al., 2019). Alternatively, the peptide, analog or derivative thereof can bind to the at least one methylation site within the 5 ' region upstream from the transcription start of the hypocretin neuropeptide precursor (HCRT) gene making it inaccessible for DNA methyltransferases, resulting in unmethylated regulatory regions and gene activation. The peptide can be endogenic, food-derived, found in the environment (microbes, fungi, etc...) or synthetic.

A peptide, an analog, or derivative thereof, when synthetic, is preferably synthesized using a chemical method known to the skilled artisan (e.g. solid phase, liquid phase, or peptide condensation, or any combination thereof), and can include natural and/or unnatural amino acids. Amino acids used for peptide synthesis may be standard Boc (Nα-amino protected Nα-t-butyloxycarbonyl) amino acid resin with the deprotecting, neutralization, coupling and wash protocols of the original solid phase procedure of Merrifield, J. Am. Chem. Soc., 85:2149-2154, 1963, or the base-labile Nα-amino protected 9-fluorenylmethoxycarbonyl (Fmoc) amino acids described by Carpino and Han, J. Org. Chem., 37:3403-3409, 1972. Both Fmoc and Boc Nα-amino protected amino acids can be obtained from various commercial sources, such as, for example, Fluka, Bachem, Advanced Chemtech, Sigma, Cambridge Research Biochemical, Bachem, or Peninsula Labs.

The peptide, analog, or derivative thereof, of the invention may be conjugated to a cell membrane permeable carrier either directly or by a spacer (e.g. via one, or more glycines). In this case the cell membrane permeable carrier is preferably a peptide, most preferably a positively charged amino acid rich peptide such as arginine rich peptides. Usually arginine rich peptides are selected from the group comprising the HIV-TAT 48-57 peptide, the FHV-coat 35 - 49 peptide, the HTLV-II Rex 4-16 peptide and the BMV gag 7-25 peptide. Preferably, the arginine rich peptide is HIV-TAT 48-57 peptide.

As used herein, the terms "nucleic acid", "polynucleotide," and "oligonucleotide" are used interchangeably and refer to any kind of deoxyribonucleotide (e.g. DNA, cDNA, ...) or ribonucleotide (e.g. RNA, miRNA, siRNA, piRNA, hnRNA, snRNA, sgRNA, esiRNA, shRNA, antisense oligonucleotide, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are chemically modified in the base, sugar and/or phosphate moieties.

The nucleic acid of the invention is selected from the group comprising, or consisting of, an acid nucleic encoding an miRNA, an siRNA, a piRNA, an hnRNA, an snRNA, an sgRNA, an esiRNA, an shRNA, peptide, an analog, or derivative thereof of the invention, and an antisense oligonucleotide (e.g. antisense oligonucleotides targeting DNMT enzymes such as MG98), or a combination thereof.

Chemical modifications of said nucleic acid or nucleotides can comprise modifications to the nucleobases, the backbone residues, and/or the internucleoside linkers of said oligonucleotides.

Modifications to one or more nucleobases of said oligonucleotides may comprise one or more alkylated purines and pyrimidines, acylated purines and pyrimidines, and other heterocycles. These classes of pyrimidines and purines are known in the art and include pseudoisocytosine, N4,N4-ethanocytosine, 8-hydroxy-N-6-methyladenine, 4-acetylcytosine, 5-(carboxyhydroxylmethyl)uracil, 5 fluorouracil, 5-bromouracil, 5-carboxymethylaminomethyl-2-thiouracil, 5-carboxy-methylaminomethyl uracil, dihydrouracil, inosine, N6-isopentyl-adenine, 1-methyladenine, 1-methylpseudouracil, 1-methylguanine, 2,2-dimethylguanine, 2-methyladenine, 2-methylguanine, 3-methylcytosine, 5-methylcytosine, N6-methyladenine, 7-methylguanine, 5-methylaminomethyl uracil, 5-methoxy amino methyl-2-thiouracil, -D-mannosylqueosine, 5-methoxycarbonylmethyluracil, 5-methoxyuracil, 2 methylthio-N-6-isopentenyladenine, uracil-5-oxyacetic acid methyl ester, psueouracil, 2-thiocytosine, 5-methyl-2 thiouracil, 2-thiouracil, 4-thiouracil, 5-methyluracil, N-uracil-5-oxyacetic acid methylester, uracil 5-oxyacetic acid, queosine, 2-thiocytosine, 5-propyluracil, 5-propylcytosine, 5-ethyluracil, 5-ethylcytosine, 5-butyluracil, 5-pentyluracil, 5-pentylcytosine, and 2,6,diaminopurine, methylpsuedouracil, 1 -methylguanine and 1-methylcytosine.

Modifications to one or more backbone residues of said nucleic acids or oligonucleotides may comprise one or more of the following: 2' sugar modifications such as 2'-O-methyl (2'-OMe), 2'-O-methoxyethyl (2'-MOE), 2'-O-methoxyethoxy, 2'-Fluoro (2'-F), 2'-Allyl, 2'-O-[2-(methylamino)-2-oxoethyl], 2'-O-(N-methylcarbamate); 4' sugar modifications including 4'-thio, 4'-CH2-O-2'-bridge, 4-(CH2)2-O-2'-bridge; Locked Nucleic Acid (LNA); Peptide Nucleic Acid (PNA); Intercalating nucleic acid (INA); Twisted intercalating nucleic acid (TINA); Hexitol nucleic acids (HNA); arabinonucleic acid (ANA); cyclohexane nucleic acids (CNA); cyclohexenylnucleic acid (CeNA); threosyl nucleic acid (TNA); Morpholino oligonucleotides; Gap-mers; Mix-mers; Incorporation Arginine-rich peptides; addition of 5'-phosphate to synthetic RNAs; RNA Aptamers; or any combinations thereof.

Modifications to one or more internucleoside linkers of said oligonucleotides may comprise one or more of the following: Phosphorothioate, phosphoramidate, phosphorodiamidate, phosphorodithioate, phosphoroselenoate, phosphorodiselenoate, phosphoroanilothioate and phosphoranilidate, or any combinations thereof.

In general, an analogue of a particular nucleotide has the same base-pairing specificity; i.e., an analogue of A will base-pair with T. For example, a nucleic acid according to the invention, can be modified to enhance expression and reduce possible toxicity by including one or more modified nucleoside e.g. using pseudo-U or 5-Methyl-C.

The terms "microRNA," "miRNA," and "MiR" are interchangeable and refer to endogenous or artificial non-coding RNAs that are capable of regulating gene expression. It is believed that miRNAs function via RNA interference.

The terms "siRNA" and "short interfering RNA" are interchangeable and refer to single-stranded or double-stranded RNA molecules that are capable of inducing RNA interference. SiRNA molecules typically have a duplex region that is between 18 and 30 base pairs in length.

The terms "piRNA" and "Piwi-interacting RNA" are interchangeable and refer to a class of small RNAs involved in gene silencing. PiRNA molecules typically are between 26 and 31 nucleotides in length.

The terms "snRNA" and "small nuclear RNA" are interchangeable and refer to a class of small RNAs involved in a variety of processes including RNA splicing and regulation of transcription factors. The subclass of small nucleolar RNAs (snoRNAs) is also included. The term is also intended to include artificial snRNAs.

As used herein, an "antibody" is a protein molecule that reacts with a specific antigenic determinant or epitope and belongs to one or five distinct classes based on structural properties: IgA, IgD, IgE, IgG and IgM. The antibody may be a polyclonal (e.g. a polyclonal serum) or a monoclonal antibody, including but not limited to fully assembled antibody, single chain antibody, antibody fragment, and chimeric antibody, humanized antibody as long as these molecules are still biologically active and still bind to at least one peptide of the invention. Preferably the antibody is a monoclonal antibody. Preferably also the monoclonal antibody will be selected from the group comprising the IgG1, IgG2, IgG2a, IgG2b, IgG3 and IgG4 or a combination thereof. Most preferably, the monoclonal antibody is selected from the group comprising the IgG1, IgG2, IgG2a, and IgG2b, or a combination thereof. The antibody of the invention is preferably an anti- DNMT enzyme antibody that acts as an inhibitor of the activity of said DNMT enzyme.

An "antigen binding fragment" comprises a portion of a full-length antibody. Examples of antigen binding fragments include Fab, Fab', F(ab')2, and Fv fragments; diabodies; minobodies; nanobodies; linear antibodies (Zapata et al. (1995) Protein Eng. 8(10):1057-1062); single-chain antibody molecules; and multispecific antibodies formed from antibody fragments.

The present invention also contemplates a gene delivery vector, preferably in the form of a plasmid or a vector, that comprises, or consists of, one or more nucleic acid(s) encoding an miRNA, a siRNA, a piRNA, a hnRNA, a snRNA, a esiRNA, a shRNA, or an antisense oligonucleotide, or a combination thereof, of the present invention. As used herein, a "vector" is capable of transferring nucleic acid sequences to target cells and is, preferably, selected from the group comprising, or consists of, viral vectors, non-viral vectors, particulate carriers, and liposomes, or a combination thereof.

Suitable vectors include derivatives of SV40 and known bacterial plasmids, e. g., E. coli plasmids col El, pCR1, pBR322, pMB9 and their derivatives, plasmids such as RP4; phage DNAs, e. g., the numerous derivatives of phage X, e. g., NM989, and other phage DNA, e. g., M1 3 and filamentous single stranded phage DNA; yeast plasmids such as the 2µ plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like.

Various viral vectors are used for delivering nucleic acid to cells in vitro or in vivo. Non-limiting examples are vectors based on Herpes Viruses, Pox- viruses, Adeno-associated virus, Lentivirus, and others. In principle, all of them are suited to deliver the expression cassette comprising an expressible nucleic acid molecule that codes for a miRNA, a siRNA, a piRNA, a hnRNA, a snRNA, a esiRNA, a shRNA, and an antisense oligonucleotide of the invention. In a preferred aspect, said viral vector is an adenoviral vector, preferably a replication competent adenovirus.

Alternatively, the gene delivery vector of the invention, preferably a viral vector is issued for delivering a CRISPR-based system comprising i) at least one sgRNA, or crRNA and tracrRNA, targeting a regulatory sequence within the 5 ' region upstream from the transcription start site of the human hypocretin neuropeptide precursor (HCRT) gene, ii) a structure-guided endonuclease such as an RNA-guided endonuclease, and and ii) a structure-guided including inactivate Cas9 with DNA methylation modification enzyme DNMT or Tet (dCas9-DNMT/Tet).

As described herein, the 5' region upstream from the transcription start site of the human hypocretin neuropeptide precursor (HCRT) gene comprises one or more regulatory elements associated with said HCRT gene. Preferably, said at least one methylation site is in a CpG region located between base pairs -238 and -239 upstream of the transcription start site of the human hypocretin neuropeptide precursor (HCRT) gene. Most preferably, the CpG region is in a Pax5-Ets1 binding site (ccggag) that will be targeted by the at least one sgRNA, or crRNA and tracrRNA.

Any suitable naturally occurring, or engineered, RNA-guided endonuclease can be employed as long as it is effective for specifically binding the regulatory sequence within the 5' region upstream from the transcription start site of the human hypocretin neuropeptide precursor (HCRT) gene of the invention and it may be selected from the non-limiting group comprising Cas9, Cpfl, and FEN-1. Preferably, the RNA-guided endonuclease is a catalytically dead RNA-guided endonuclease, most preferably a catalytically dead Cas9 (dCas9).

More preferably, the catalytically dead RNA-guided endonuclease such as, e.g. the dCas9, is fused to one or more DNA methylation modification enzymes such as, e.g. Dnmt or Tet, allowing for targeted modification of DNA methylation (as described, e.g. in Xu et al., 2016 and Liu et al., 2019, which disclosures are incorporated herein by reference)

Also contemplated in the present invention is a host cell comprising a plasmid or vector of the invention or one or more nucleic acid(s) encoding the miRNA, siRNA, piRNA, hnRNA, snRNA, sgRNA, esiRNA, shRNA, and/or antisense oligonucleotide of the invention. The host cell can be any prokaryotic or eukaryotic cell, preferably the host cell is a eukaryotic cell, most preferably the host cell is a mammalian cell. Even more preferably, the host cell is a human neuronal cell, a glial cell and/or a stem cell (e.g. iPSCs).

The invention further provides pharmaceutical compositions comprising a therapeutically effective amount of i) an agent modulating the level of methylation of at least one methylation site within the 5 ' region upstream from the transcription start site of the human hypocretin neuropeptide precursor (HCRT) gene, or ii) a plasmid or a vector of the invention, or iii) a host cell of the invention, and a pharmaceutically acceptable carrier or diluent.

Preferably, the agent is a chemical compound, a peptide or analog thereof, an antibody or an antigen-binding fragment of said antibody or a nucleic acid, as described herein.

The term " therapeutically effective amount" as used herein means an amount of a pharmaceutical composition of the invention high enough to significantly positively modify the symptoms and/or condition to be treated, but low enough to avoid serious side effects (at a reasonable risk/benefit ratio), within the scope of sound medical judgment.

The therapeutically effective amount of the pharmaceutical composition of the invention is selected in accordance with a variety of factors including type, species, age, weight, sex and medical condition of the patient; the severity of the condition to be treated; the route of administration; the renal and hepatic function of the patient. A physician of ordinary skill in the art can readily determine and prescribe the effective amount of the drug required to prevent, counter or arrest the progress of a neurological disease, a neuropsychiatric disease, a neurodegenerative disease, and associated diseases or disorders, preferably narcolepsy.

The therapeutically effective amount of the pharmaceutical composition of the invention as described herein, will vary depending upon the subject treated and the particular mode of administration. For example, a time-release formulation intended for oral administration to humans may contain approximately 0.10 to 500 mg of active material compounded with an appropriate and convenient amount of carrier material which may vary from about 5 to about 95% of the total compositions. It is preferred that the pharmaceutical composition be prepared which provides easily measurable amounts for administration. Typically, a therapeutically effective amount to be administered systemically is about 0.1 mg/kg to about 100 mg/kg and depends upon a number of factors including, for example, the age and weight of the subject (e.g., a mammal such as a human), the precise condition requiring treatment and its severity, the route of administration, and will ultimately be at the discretion of the attendant physician or veterinarian. The therapeutically effective amount of the pharmaceutical composition of the invention, as described herein, will also vary depending upon the acetylation phenotype of the patient.

Preferably, the subject will be administered one or more doses per day, week, or month, such as daily, for example, from 1 to 10 times daily, or more (e.g., 1 , 2, 3, 4, 5, 6, 7, 8, 9, 10, or more, times daily). The administration will be stopped once the clinical symptoms are arrested and/or the level and/or concentration of hypocretin (type 1 and/or type 2), when compared to a control sample or reference range, is considered normal. Any method capable of determining the level and/or concentration of hypocretin (type 1 and/or type 2) is contemplated by this disclosure.

Suitable routes of administration of the pharmaceutical composition of the invention include parenteral administration, such as subcutaneous (SC), intraperitoneal (IP), intramuscular (IM), intravenous (IV), or infusion, oral and pulmonary, nasal, topical, transdermal, and suppositories. Where the composition is administered via pulmonary delivery, the therapeutically effective dose is adjusted such that the soluble level of the agent is equivalent to that obtained with a therapeutically effective dose that is administered parenterally, for example SC, IP, IM, or IV. In some preferred aspects of the invention, the pharmaceutical composition is administered by IV or nasal delivery.

In other aspects of the invention, the pharmaceutical composition of the invention is a i) sustained-release formulation, or a formulation that is administered using a sustained-release device, ii) a controlled release formulation or iii) a slow release formulation. Sustained-release devices are well known in the art, and include, for example, transdermal patches, and miniature implantable pumps that can provide for drug delivery over time in a continuous, steady-state fashion at a variety of doses to achieve a sustained-release effect with a non-sustained-release pharmaceutical composition. Controlled release formulations as well as slow release formulations are also well known in the art.

"Pharmaceutically acceptable carrier or diluent" means a carrier or diluent that is useful in preparing a pharmaceutical composition that is generally safe, non-toxic, and desirable, and includes carriers or diluents that are acceptable for human pharmaceutical use.

Such pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions.

Pharmaceutically acceptable excipients include starch, glucose, lactose, sucrose, sodium stearate, glycerol monostearate, talc, sodium chloride, dried skim milk, glycerol, propylene glycol, water, ethanol and the like.

Also contemplate are pharmaceutical compositions that are formulated so as to deliver the agent of the invention to the brain across the blood-brain barrier (BBB). These pharmaceutical compositions may comprise e.g. i) nanocarriers and nanoparticles made, e.g. of poly(butyl cyanoacrylate) (PBCA) or poly(lactic-co-glycolic acid) (PLGA) coated with polysorbate 80 or poloxamer 188 to enable the transport of a chemical compound of the invention, ii) viral vectors to enable the transport of a nucleic acid of the invention, iii) exosomes or liposomes, iv) molecular vectors - also known as BBB shuttles (e.g. Trojan horse antibodies), or v) the peptide or analog of the invention conjugated to PEG and/or a cell membrane carrier as described herein.

The pharmaceutical compositions may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include macrocrystalline cellulose, carboxymethyf cellulose sodium, polysorbate 80, phenyletbyl alcohol, chiorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991) which is incorporated by reference herein.

In one aspect, the pharmaceutical composition further comprises one or more histone deacetylase (HDAC) agent that can be administered concomitantly, separately or staggered. The histone deacetylase agent is usually selected from the group comprising, or consisting of, a chemical compound, a peptide or analog thereof and a nucleic acid (see for example Janssens et al. 2019 and Hauser et al., 2018, which disclosure is incorporated herein by reference). In case the histone deacetylase agent is a non-peptidyl chemical compound, it will be elected among the non-limiting group comprising, or consisting of, valproic acid, sodium butyrate, nicotinamide, Vorinostat, Trichostatin, Givinostat, and similar agents, or a combination thereof.

A peptide or analog thereof having histone deacetylase properties will be selected among the non-limiting group comprising, or consisting of, Romidepsin, Burkholdacs, Spiruchostatins, Thailandepsin, FR901375, Largazole, Plitidepsin, Chlamydocin, Trapoxins, CHAP, Apidicin, Microsporins, Azumamides, FR235222 and AS 1387392, or a combination thereof.

The invention also provides the use of compositions and pharmaceutical compositions of the invention in the preparation of a medicament for the treatment and/or prevention of a neurological disease, a neuropsychiatric disease, a neurodegenerative disease, preferably narcolepsy and associated diseases or disorders.

The invention also provides a method of treating and/or preventing a neurological disease, a neuropsychiatric disease, a neurodegenerative disease, preferably narcolepsy and associated diseases or disorders, in a subject in need thereof, the method comprising administering a therapeutically-effective amount of one or more pharmaceutical compositions to the subject, the one or more pharmaceutical compositions comprising i) an agent modulating the level of methylation of at least one methylation site within the 5' region upstream from the transcription start site of the human hypocretin neuropeptide precursor (HCRT) gene, or ii) a plasmid or a vector of the invention, or iii) a host cell of the invention, and a pharmaceutically acceptable carrier or diluent.

In one aspect, the method of treating and/or preventing further comprises administering a therapeutically-effective amount of a pharmaceutical composition comprising one or more histone deacetylase (HDAC) agent.

The compositions or pharmaceutical compositions of the invention, are administered concomitantly, separately or staggered.

Combined or concomitant administration can include co-administration, either in a single pharmaceutical formulation or using separate formulations, or consecutive administration in either order but generally within a time period such that all active agents can exert their biological activities simultaneously. Preparation and dosing schedules for such agents can be used according to manufacturers' instructions or as determined empirically by the skilled practitioner.

The present disclosure further discloses a method for detecting a neurological disease, a neuropsychiatric disease, a neurodegenerative disease, preferably narcolepsy, and associated diseases or disorders, in a biological sample which comprises (a) determining the level and/or concentration of hypocretin (type 1 and/or type 2), wherein a low level and/or low concentration of said hypocretin (type 1 and/or type 2), when compared to a control sample, is indicative of the subject being susceptible to develop or to have a neurological disease, a neuropsychiatric disease, a neurodegenerative disease, preferably narcolepsy and associated diseases or disorders.

The method further comprises a step of (c) administering at least one pharmaceutical composition comprising a therapeutically effective amount of i) an agent modulating the level of methylation of at least one methylation site within the 5 ' region upstream from the transcription start site of the human hypocretin neuropeptide precursor (HCRT) gene as described herein, or ii) a plasmid or a vector of the invention, or iii) a host cell of the invention, and a pharmaceutically acceptable carrier or diluent. As used herein, a "biological sample" refers to a sample of a cell or cells, tissue, or fluid isolated from a subject, including but not limited to, for example, blood, plasma, serum, fecal matter, urine, bone marrow, bile, cerebrospinal fluid, lymph fluid, samples of the skin, external secretions of the skin, respiratory, intestinal, and genitourinary tracts, tears, saliva, milk, blood cells, organs, biopsies and also samples of in vitro cell culture constituents including but not limited to conditioned media resulting from the growth of cells and tissues in culture medium, e.g., recombinant cells, and cell components. Preferably, the biological sample is a fluid, most preferably the biological sample is cerebrospinal fluid.

The present disclosure also discloses a computer implemented method for detecting the neurological disease, neuropsychiatric disease, neurodegenerative disease, preferably narcolepsy, and associated diseases or disorders, as described above.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

### EXAMPLES

### Material & Methods

### Brain tissue, DNA preparation

Formalin fixed sections (2-4 sections /subject) through the lateral hypothalamus from 7 narcolepsy patients and 7 controls were obtained through international collaborators (4 patients and 4 controls from USA, 2 patients and 2 controls from France, and 1 patient and 1 control from the Netherlands). Sections were washed with PBS and genomic DNA was extracted with AIAmp DNA FFPE Tissue kit (Qiagen).

### DNA methylation analysis

Five hundred ng of DNA was treated with sodium bisulfite using the EpiTect Fast DNA Bisulfite kit (Qiagen). Primers for nested PCR (2 fragments for *HCRT*, 1 for *PDYN*) were designed with MethPrimer (Li and Dahiya, 2002) (Table 1).

**Table 1. Bisulfite primers used in this study.**

| ***Hcrt*** | | **SEQ ID No.** |
|---|---|---|
| First fragment: F1 | GAAGAAGGTTTTGGAGTTTGATAGTT | 2 |
| First fragment: R1 | CTATACCCCTAATCACCCCCTTAT | 3 |
| First fragment: F2 | TTGGGTGTAAGGTGGTTTTATTAGT | 4 |
| First fragment: R2 | TTATAACCCACTCCCAAAAATCTAA | 5 |
| Second fragment: F1 | GGATTGTTGTTGGTTGTTTTATTTT | 6 |
| Second fragment: R1 | CTATACCCCTAATCACCCCCTTAT | 7 |
| Second fragment: F2 | ATAAGGGGGTGATTAGGGGTATAG | 8 |
| Second fragment: R2 | TTATAACCCACTCCCAAAAATCTAA | 9 |

| ***Pdyn*** | | |
|---|---|---|
| F1 | GTTGTGTTTTTGTTAGGGTTAGTGT | 10 |
| R1 | CAAAAACTAAAACAATCTTCTAATCAC | 11 |
| F2 | GTTTGGTATTAGTTTAGGTATGTATTAGAG | 12 |
| R2 | CAAAAACTAAAACAATCTTCTAATCAC | 13 |

| ***Hcrtr2*** | | |
|---|---|---|
| F1 | GGTGTTATTGTTGTAGTTTTTAGTGT | 14 |
| R1 | AACTCTTAAATTTCATTCAACTCC | 15 |
| F2 | GAGGTATTGGTTTAGTAATTTTTTA | 16 |
| R2 | AACTCTTAAATTTCATTCAACTCC | 17 |

Two methods were used to quantify CpG methylation. In the first method PCR amplicons were gel purified followed by Sanger sequencing. Sequence trace files were analyzed by ESME software (Lewin et al., 2004) resulting in average methylation at each CpG. In the second method purified PCR amplicons (100 ng) were used to prepare sequencing libraries the TruSeq Nano DNA LT Library Preparation Kit (Illumina) with the following modifications. Purified bisulfite PCR products were not sheared, but directly subjected to end repair and then cleaned up with a single round of purification beads at a 1.8X ratio. The standard protocol was strictly followed for the subsequent steps. Libraries were quantified with a fluorimetric method (QubIT, Thermo Fischer) and their size pattern evaluated with the Fragment Analyzer (Agilent). An equimolar pool of each libraries was prepared and loaded at 10 pM (with 25% PhiX spike in for generating sequencing diversity) for a 150 cycles paired end run on a MiSeq instrument (Illumina) using a v2 reagent kit. Sequencing data were demultiplexed using the bcl2fastq conversion software (version 2.20, Illumina). Quality control was performed to keep only read-pairs that could be reconstructed (between 59 and 67% of the reads). On average, 287,852 reads (range 219,476-349,712 reads) were obtained par subject. A weight matrix profile was prepared from the reference sequence to take into account the C to T alterations caused by bisulfite treatment and the reads were aligned to the profile using pfsearch from pftools. A summary table counting the number of occurrences of each nucleotides along the fragment of interest was produced and the ratio of methylated C nucleotides was computed as a percentage of methylation for each position.

### Hcrt promotor cloning and site directed mutagenesis

1800 base pairs of the human *Hcrt* promoter upstream of the transcription start site, were cloned in multiple cloning site of the pCpG-basic vector (gift from Dr. Michael Rehli lab) upstream of Firefly luciferase sequence. Single nucleotide mutagenesis was performed according to Q5® Site-Directed Mutagenesis Kit (New England Biolabs, E0552s). The mutated plasmids were transformed in Pir1 bacterial strain and sequenced. The confirmed colonies were cultured at large scale and maxi-plasmid preparations were carried out using HiSpeed Plasmid Maxi Kit (Qiagen, 12662). CpG Methyltransferase, M.SssI (New England Biolabs, M0226s) was used to methylate the CpG sites on the *Hcrt* promoter.

### Luciferase assay

The primary mouse hypothalamic cells and cell lines were cultured in 24 well pates and the day after were transfected with methylated and non-methylated plasmids. The transfection was performed using X-tremeGENE™ HP DNA Transfection Reagent (Sigma, 06 366 244 001) with the DNA to reagent ration of 1:3. The transfected plasmids were mixed with Firefly and Renilla luciferases with ratio of 19:1. 48 hours after transfection the cells were lysed and prepared for the luciferase activity measurement according to Promega kit (E1910). Luciferase activity was measured using glomax 96 microplate luminometer from Promega. The Firefly luciferase activity was normalized to Renilla activity.

### Results

### CpG methylation

We have used bisulfite sequencing and two methods were employed to determine CpG methylation. The first method employs direct Sanger sequencing of PCR amplicons, which measures the average methylation of all DNA molecules in a tissue. The second method employs high throughput single DNA molecule sequencing. 12 of the 13 CpG sites (all except the first CpG in Fig. 1) were tested. Eight of the 12 CpGs showed higher methylation in narcoleptics and one was significantly hypermethylated (position 5, methylation 93.9% in narcoleptics and 69.93% in controls, p<0.01).

The hypermethylated site is indicated in bold in Fig. 1. This CpG is within a Pax5-Etsl binding site (Pax5-EBS) (Fig. 1). Pax5 recruits Etsl at the Ets suboptimal (5'-CCGGAG-3') binding site and is essential in the regulation of B cell-specific expression of the mb-1 gene.

### Post-mortem analysis

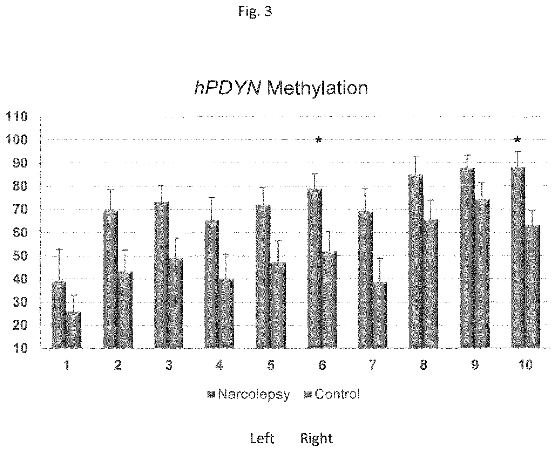

Post-mortem analysis of narcolepsy patients indicated that two other neuropeptide PDYN (Dynorphin) and NPTX2 (Narp), which colocalize with HCRT are also lost in the lateral hypothalamus. Interestingly, PDYN and NARP, as HCRT gene, have a CpG island in their promoters, suggesting that the hypermethylation of HCRT neurons may silence other colocalized genes with CpG rich promoters. NPTX2 promoter contains a very high density CpG island which makes bisulfite sequencing very difficult. We therefore tested the methylation of PDYN promoter by bisulfite sequencing. All 10 CpGs tested were more methylated in narcoleptics than in controls and 2 (position 6 and 10) were significantly hypermethylated (Fig. 3).

### Effect of methylation on gene transcription

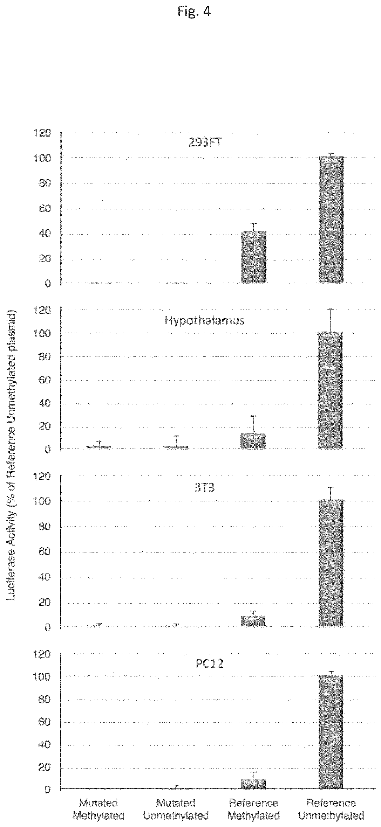

To test the effect of methylation on gene transcription we performed luciferase assays. The 1800bp of human proximal *Hcrt* promoter was cloned in a CpG-Free luciferase reporter vector (pCpGL). Four different vectors were tested: pCpGL with the human *Hcrt* promoter (Reference Unmethylated), pCpGL with the methylated (in vitro methylation by M.Sssl enzyme) human *Hcrt* promoter (Reference Methylated), pCpGL with the human *Hcrt* promoter including a C to T mutation at the critical CpG (Mutated Unmethylated), and the mutated and methylated vector (Mutated Methylated). All vectors were transfected in 3 cell lines as well as mouse primary hypothalamic cultures. Methylation of the *Hcrt* promoter decreased the expression of luciferase by 60 to 90% (p<10-7) in the 4 cell culture conditions (Fig. 4). The C/T mutation comopletely abolished luciferase expression (p<10-7), both in methylated and unmethylated vector (Fig. 4). These in vitro results confirm that *Hcrt* promoter methylation critically controls *Hcrt* expression and that the hypermethylated CpG identified is the most important site for *Hcrt* expression.

### REFERENCES

Ahuja N, Sharma AR, Baylin SB (2016) Epigenetic Therapeutics: A New Weapon in the War Against Cancer. Annu Rev Med 67:73-89.
Candelaria M, Herrera A, Labardini J, Gonzalez-Fierro A, Trejo-Becerril C, Taja-Chayeb L, Perez-Cardenas E, de la Cruz-Hernandez E, Arias-Bofill D, Vidal S, Cervera E, Duenas-Gonzalez A (2011) Hydralazine and magnesium valproate as epigenetic treatment for myelodysplastic syndrome. Preliminary results of a phase-II trial. Ann Hematol 90:379-387.
Candelaria M, Gallardo-Rincon D, Arce C, Cetina L, Aguilar-Ponce JL, Arrieta O, Gonzalez-Fierro A, Chavez-Blanco A, de la Cruz-Hernandez E, Camargo MF, Trejo-Becerril C, Perez-Cardenas E, Perez-Plasencia C, Taja-Chayeb L, Wegman-Ostrosky T, Revilla-Vazquez A, Duenas-Gonzalez A (2007) A phase II study of epigenetic therapy with hydralazine and magnesium valproate to overcome chemotherapy resistance in refractory solid tumors. Ann Oncol 18:1529-1538.
Chen YH, Huang YS, Chen CH (2013) Increased plasma level of tumor necrosis factor alpha in patients with narcolepsy in Taiwan. Sleep Med 14:1272-1276.
Dauvilliers Y, Abril B, Mas E, Michel F, Tafti M (2009) Normalization of hypocretin-1 in narcolepsy after intravenous immunoglobulin treatment. Neurology 73:1333-1334.
Dauvilliers Y, Abril B, Charif M, Quittet P, Bauchet L, Carlander B, Touchon J (2007) Reversal of symptomatic tumoral narcolepsy, with normalization of CSF hypocretin level. Neurology 69:1300-1301.
Dauvilliers Y, Montplaisir J, Cochen V, Desautels A, Einen M, Lin L, Kawashima M, Bayard S, Monaca C, Tiberge M, Filipini D, Tripathy A, Nguyen BH, Kotagal S, Mignot E (2010) Post-H1N1 narcolepsy-cataplexy. Sleep 33:1428-1430.
E de la Cruz-Hernandez, E Perez-Cardenas, L Taja-Chayeb, A Chavez-Blanco, C Trejo-Becerril, J Trujillo, JL Medina-Franco, A Dueñas-Gonzalez. DNA Demethylating Activity of Hydralazine in Cancer Cell Lines. Life Sciences and Medicine Research, Volume 2011
Espinoza-Zamora JR, Labardini-Mendez J, Sosa-Espinoza A, Lopez-Gonzalez C, Vieyra-Garcia M, Candelaria M, Lozano-Zavaleta V, Toledano-Cuevas DV, Zapata-Canto N, Cervera E, Duenas-Gonzalez A (2017) Efficacy of hydralazine and valproate in cutaneous T-cell lymphoma, a phase II study. Expert Opin Investig Drugs 26:481-487.
Hallmayer J et al. (2009) Narcolepsy is strongly associated with the T-cell receptor alpha locus. Nat Genet 41:708-711.
Hauser ATI, Robaa D2, Jung M3. Epigenetic small molecule modulators of histone and DNA methylation. Curr Opin Chem Biol. 2018 Aug;45:73-85. doi:10.1016/j.cbpa.2018.03.003.
Himmerich H, Beitinger PA, Fulda S, Wehrle R, Linseisen J, Wolfram G, Himmerich S, Gedrich K, Wetter TC, Pollmacher T (2006) Plasma levels of tumor necrosis factor alpha and soluble tumor necrosis factor receptors in patients with narcolepsy. Arch Intern Med 166:1739-1743.
Hohjoh H, Terada N, Miki T, Honda Y, Tokunaga K (2001) Haplotype analyses with the human leucocyte antigen and tumour necrosis factor-alpha genes in narcolepsy families. Psychiatry Clin Neurosci 55:37-39.
Hohjoh H, Nakayama T, Ohashi J, Miyagawa T, Tanaka H, Akaza T, Honda Y, Juji T, Tokunaga K (1999) Significant association of a single nucleotide polymorphism in the tumor necrosis factor-alpha (TNF-alpha) gene promoter with human narcolepsy. Tissue Antigens 54:138-145.
Kashiwagi M TT, Hara K, Suzuki S, Wakamiya E, Shimizu T, et al. (2004) Sleepiness due to Wernicke's encephalopathy with bilateral hypothalamic lesion in a 5-year-old girl.. Sleep Suppl:A315.
Latorre D, Kallweit U, Armentani E, Foglierini M, Mele F, Cassotta A, Jovic S, Jarrossay D, Mathis J, Zellini F, Becher B, Lanzavecchia A, Khatami R, Manconi M, Tafti M, Bassetti CL, Sallusto F (2018) T cells in patients with narcolepsy target self-antigens of hypocretin neurons. Nature 562:63-68.
Lewin J, Schmitt AO, Adorjan P, Hildmann T, Piepenbrock C (2004) Quantitative DNA methylation analysis based on four-dye trace data from direct sequencing of PCR amplificates. Bioinformatics 20:3005-3012.
Li LC, Dahiya R (2002) MethPrimer: designing primers for methylation PCRs. Bioinformatics 18:1427-1431.
Liu XS, Wu H, Krzisch M, Wu X, Graef J, Muffat J, Hnisz D, Li CH4, Yuan B, Xu C, Li Y, Vershkov D, Cacace A, Young RA, Jaenisch R - Rescue of Fragile X Syndrome Neurons by DNA Methylation Editing of the FMR1 Gene. Cell. 2018 Feb 22;172(5):979-992.e6. doi: 10.1016/j.cell.2018.01.012. Epub 2018 Feb 15.
Nishino S, Ripley B, Overeem S, Lammers GJ, Mignot E (2000) Hypocretin (orexin) deficiency in human narcolepsy. Lancet 355:39-40.
Nozaki H, Shimohata T, Kanbayashi T, Sagawa Y, Katada S, Satoh M, Onodera O, Tanaka K, Nishizawa M (2009) A patient with anti-aquaporin 4 antibody who presented with recurrent hypersomnia, reduced orexin (hypocretin) level, and symmetrical hypothalamic lesions. Sleep Med 10:253-255.
Oka Y, Kanbayashi T, Mezaki T, Iseki K, Matsubayashi J, Murakami G, Matsui M, Shimizu T, Shibasaki H (2004) Low CSF hypocretin-1/orexin-A associated with hypersomnia secondary to hypothalamic lesion in a case of multiple sclerosis. J Neurol 251:885-886.
Peyron C et al. (2000) A mutation in a case of early onset narcolepsy and a generalized absence of hypocretin peptides in human narcoleptic brains. Nat Med 6:991-997.
Pérez-Cárdenas E, Taja-Chayeb L, Trejo-Becerril C, Chanona-Vilchis J, Chávez-Blanco A, Domínguez-Gómez G, Langley E, García-Carrancá A, Dueñas-González A Antimetastatic effect of epigenetic drugs, hydralazine and valproic acid, in Ras-transformed NIH 3T3 cells. Onco Targets Ther. 2018 Dec 7;11:8823-8833. doi: 10.2147/OTT.S187306. eCollection 2018.
Schroeder NA (1952) The effect of 1-hydrasinophthalasine in hypertension. Circulation 5:28-37.
Tafti M et al. (2014) DQB1 locus alone explains most of the risk and protection in narcolepsy with cataplexy in Europe. Sleep 37:19-25.
Tampe B, Tampe D, Zeisberg EM, Muller GA, Bechtel-Walz W, Koziolek M, Kalluri R, Zeisberg M (2015) Induction of Tet3-dependent Epigenetic Remodeling by Low-dose Hydralazine Attenuates Progression of Chronic Kidney Disease. EBioMedicine 2:19-36.
Vassalli A, Li S, Tafti M (2015) Comment on "Antibodies to influenza nucleoprotein cross-react with human hypocretin receptor 2". Sci Transl Med 7:314le312.
Xu X, Tao Y, Gao X, Zhang L, Li X, Zou W, Ruan K, Wang F, Xu GL, Hu R.A CRISPR-based approach for targeted DNA demethylation. (2016) Cell Discov. May 3
Zambrano P, Segura-Pacheco B, Perez-Cardenas E, Cetina L, Revilla-Vazquez A, Taja-Chayeb L, Chavez-Blanco A, Angeles E, Cabrera G, Sandoval K, Trejo-Becerril C, Chanona-Vilchis J, Duenas-Gonzalez A (2005) A phase I study of hydralazine to demethylate and reactivate the expression of tumor suppressor genes. BMC Cancer 5:44.

## Claims

1. An agent for use in the treatment and/or prevention of narcolepsy, wherein the agent modulates the level of methylation of at least one methylation site within the 5 ' region upstream from the transcription start site of the human hypocretin neuropeptide precursor (HCRT) gene.

2. The agent for use of claim 1, wherein the agent decreases the level of methylation of said at least one methylation site within the 5 ' region upstream from the transcription start site of the human hypocretin neuropeptide precursor (HCRT) gene.

3. The agent for use of claim 1 or 2, wherein said 5' region upstream from the transcription start site of the human hypocretin neuropeptide precursor (HCRT) gene comprises one or more regulatory elements associated with said HCRT gene.

4. The agent for use of anyone of claims 1 to 3, wherein said at least one methylation site is in a CpG region located between base pairs -238 and -239 upstream of the transcription start site of the human hypocretin neuropeptide precursor (HCRT) gene.

5. The agent for use of claim 4, wherein the CpG region is in a Pax5-Ets1 binding site (ccggag).

6. The agent for use of anyone of claims 1 to 5, wherein the agent is selected from the group comprising a chemical compound, a peptide or analog thereof, an antibody or an antigen-binding fragment of said antibody and a nucleic acid, or a combination thereof.

7. The agent for use of claim 6, wherein the chemical compound possesses DNA-demethylating properties.

8. The agent for use of claim 7, wherein the chemical compound possessing DNA-demethylating properties is selected from the group comprising nucleoside DNMT inhibitors (such as e.g. 5-azacytidine (Vidaza), 5-aza-2'-deoxycytidine (Decitabine or Dacogen), 5-fluoro-2'-deoxycytidine, and zebularine), non-nucleoside inhibitors of DNMT (e.g. antisense oligonucleotides targeting DNMT enzymes such as MG98), Hydrazinophthalazines-derivates (hydralazine and dihydralazine), polyphenol (-)-epigallocatechin-3-gallate (EGCG), and procainamide, or a combination thereof.

9. The agent for use of claim 6, wherein the peptide or analog thereof is able to modulate epigenetic mechanisms and is selected from the group comprising XXX or a combination thereof.

10. The agent for use of claim 6, wherein the nucleic acid is selected from the group comprising an acid nucleic encoding an miRNA, an siRNA, a piRNA, an hnRNA, an snRNA, an sg RNA, an esiRNA, an shRNA, and an antisense oligonucleotide (such as e.g. antisense oligonucleotides targeting DNMT enzymes such as MG98), a variant, a fragment or a combination thereof.

11. A plasmid or a vector comprising one or more nucleic acid(s) encoding the miRNA, siRNA, piRNA, hnRNA, snRNA, sgRNA, esiRNA, shRNA, and/or antisense oligonucleotide of claim 10.

12. A host cell comprising a plasmid or vector of claim 11 or one or more nucleic acid(s) encoding the miRNA, siRNA, piRNA, hnRNA, snRNA, sgRNA, esiRNA, shRNA, and/or antisense oligonucleotide of claim 10.

13. A pharmaceutical composition comprising a therapeutically effective amount of
i) an agent modulating the level of methylation of at least one methylation site within the 5 ' region upstream from the transcription start site of the human hypocretin neuropeptide precursor (HCRT) gene, or
ii) a plasmid or a vector of claim 11, or
iii) a host cell of claim 12,
and a pharmaceutically acceptable carrier or diluent.

14. The pharmaceutical composition of claim 13, wherein the agent is a chemical compound, a peptide or analog thereof, an antibody or an antigen-binding fragment of said antibody or a nucleic acid.

15. The pharmaceutical composition of claim 13 or 14, further comprising one or more deacetylase agent.
